# EUROPEAN PATENT APPLICATION

(11) **EP 3 791 865 A1**
(43) Date of publication of application: **17.03.2021**
(21) Application number: 19197467.4
(22) Date of filing: 16.09.2019
(51) Int. Cl.: A61K 9/16, A61K 9/50, A61K 9/51, A61K 31/337

(54) **ACTIVE SUBSTANCE DELIVERY SYSTEM WITH DELAYED DELIVERY**

(71) Applicant: CAPNOMED GmbH, 78658 Zimmern (DE)
(72) Inventor: Sahoo, Ranjita, 47574 Goch (DE)
(74) Representative: Eisenführ Speiser

(57) **Abstract**

The present invention relates to an active substance delivery system, preferably an anti-cancer agent delivery system, for use in the treatment of cancer in a human subject, comprising one or more anti-cancer agents and optionally further active substances, both included in nano- and/or microparticles, and a method for producing such a delivery system.

## Description

The present invention relates to an active substance delivery system for use in the treatment of cancer in a human subject, a mixture for use in the treatment of cancer comprising such a delivery system and a method for producing such a delivery system.

Cancer is the second leading cause of death globally, and is responsible for an estimated 9.6 million deaths in 2018. Globally, about 1 in 6 deaths is due to cancer. Among the most common cancers are lung cancer (2.09 million cases, 1.76 million deaths in 2018), breast cancer (2.09 million cases, 627,000 deaths in 2018), colorectal cancer (1.80 million cases, 862,000 deaths in 2018), prostate cancer (1.28 million cases) and stomach cancer (1.03 million cases, 783,000 deaths in 2018).

Metastases are new pathological sites spread by a cancerous (e.g. malignant) tumour. Malignant tumours are capable to invade into adjacent and distant tissue, e.g. by invasion into the blood circulation, followed by invasion into another site, where another tumour (i.e. a metastasis) may grow. Likewise, such tumours may spread along various tissues, which complicates especially a surgical removal of the tumour.

The cancer site as used herein is the part or cavity of the body, i.e. the respective tissue or part of a tissue, where cancer cells located. Cavities also include hollow organs. Often, hollow organs such as the oesophagus, the stomach, the urinary bladder or other organs e.g. containing a virtual space and lined with an epithelium may serve as cancer site.

Particularly in view of cancer sites, the abdominal cavity and the pleural cavities are important regions of the body. The abdomen stretches from the thorax at the thoracic diaphragm to the pelvis at the pelvic brim. The region occupied by the abdomen is termed the abdominal cavity and contains many organs such as the stomach, the small intestine and the colon, the liver, the gall bladder and the pancreas. The abdominal cavity and the pleural cavities are coated by an extensive membrane, called the peritoneum or, respectively, the pleura, which covers the abdominal wall and the pelvic walls (parietal peritoneum) as well as the included organs (visceral peritoneum) or, respectively the inner surface of the thoracic cavity (parietal pleura) as well as the included organs (visceral pleura).

A special form of metastases are abdominal, particularly peritoneal metastases. As the cancer cells are attached to the outside of several organs and tissues but reach into the area of the peritoneum (and are thus classified as abdominal or particularly peritoneal or, respectively, pleural), these metastases are more difficult to reach with medication via the blood circulation. Cancer cells may therefore "escape" into the peritoneum and/or peritoneal cavity. Still, in many cases, these cancer sites are covered by the peritoneum, i.e. are positioned between the respective organ or tissue and the peritoneum or the pleura.

The terms "peritoneal metastasis" and "peritoneal cancer" as used herein may be understood as synonyms. Both terms comprise cancer cells of a primary or a secondary site of the host's body.

The terms "pleural metastasis" and "pleural cancer" as used herein may be understood as synonyms. Both terms comprise cancer cells of a primary or a secondary site of the host's body.

The treatment options for cancer strongly depend on several factors such as the involved organs, the cause of cancer and further complicating factors as well as the age and health status of the patient.

Although many limitations, for various cancer types, a systemic chemotherapy is applied as a standard of care. However, several tissues have a poor vascularisation and can thus be poorly reached by a systemically applied chemotherapy. The therapy thus results in a poor performance. As a counteractive measure, higher doses and/or higher volumes of the anti-cancer agent(s) could be applied transport higher amounts of the anti-cancer agent into the poorly vascularised tissues. However, increasing concentrations also increase the risk of dangerous adverse effects, renal toxicity, neurotoxicity or cardiac toxicity.

As an improvement to systemic chemotherapy, local chemotherapy can be applied to patients with tumour sites in poorly vascularized tissues. The delivery of the chemotherapeutically active substance is thereby localised to a desired tissue or region to achieve desired chemotherapeutic effect.

Such treatment options have recently emerged particularly for cancer sites in the poorly vascularized peritoneal cavity or pleural cavities:
Possible treatment options for peritoneal metastasis are e.g. Hyperthermic Intraperitoneal Chemotherapy (HIPEC) or Pressurized Intraperitoneal Aerosol Chemotherapy (PIPAC). Possible treatment options for pleural metastasis is Pressurized Intrathoracic Aerosol Chemotherapy (PITAC). In some cases, electro-precipitation may also be an option (as described for example in Kakchekeeva et al., In Vivo Feasibility of Electrostatic Precipitation as an Adjunct to Pressurized Intraperitoneal Aerosol Chemotherapy (ePIPAC), Ann Surg Oncol. 2016 Dec;23(Suppl 5):592-598 or in Reymond et al., Electrostatic precipitation Pressurized IntraPeritoneal Aerosol Chemotherapy (ePIPAC): first in-human application, Pleura Peritoneum, 2016 Jun 1;1(2):109-116).

Hyperthermic Intraperitoneal Chemotherapy (HIPEC) is a treatment usually performed after surgery when all visible cancer sites in the abdomen are surgically removed. A liquid heated chemotherapy is applied to the peritoneal cavity, bathing all reachable organs and their surfaces. With this treatment, any remaining cancer cells shall be killed. However, this treatment is a time-consuming process with higher mortality rates.

Pressurized Intraperitoneal Aerosol Chemotherapy (PIPAC) as well as Pressurized Intrathoracic Aerosol Chemotherapy (PITAC) are palliative treatments of peritoneal or, respectively, pleural metastasis. PIPAC is a method for applying chemotherapeutic drugs, in form of an aerosol, under pressure into the abdominal cavity. The aerosol is usually applied within the closed abdominal or, respectively, thoracic cavity, i.e. during a laparoscopy or, respectively, thoracoscopy. PIPAC and PITAC are significantly time saving therapies compared to other available techniques like HIPEC.

In many cases, however, sole chemotherapy is not sufficient for treating cancer and a surgery, in which the cancer cells are removed, becomes necessary or even the only remaining option. Surgeries generally provide risks to patients such as (internal) bleedings, infections and stress for the cardiovascular system during anaesthesia. Nevertheless, surgeries are usually necessary if performed. However, to prevent the risks involved, unnecessary surgeries should be avoided.

A drawback of the surgical removal of cancer cells is that in several cases, some cancer cells or cancer cell clusters remain in the body, as they were too small to be seen or detected during surgery. These cancer cells or cancer cells clusters usually tend to be very aggressive, i.e. are rapidly growing and likely to spread in form of metastases.

Furthermore, healing includes the rapid growth of tissue in proximity of the wound, which is usually triggered or accompanied by a mixture of endogenous growth stimulants. Such stimulants may also promote growth of the remaining cancer cells or cancer cell clusters.

As the body is weakened by performing the surgery, a systemic chemotherapy to kill the remaining cancer cells or cancer cell clusters is not an option. Also, local chemotherapy often cannot be performed, as the substances themselves usually interfere with the healing process or as the local application of the anti-cancer agent is performed under pressure (e.g. in case of PIPAC or PITAC) or other (physical) interactions with the tissue affected by surgery.

Moreover, even if the anti-cancer agents reach their destination, their concentration must be low enough to ensure proper wound healing but at the same time high enough to fight the remaining cancer cells or cancer cell clusters. Thus, their concentration needs to be tightly regulated over time (concentrations may and should be increased with increasing healing progress) and be analysed to intervene if it becomes necessary.

Furthermore, as any current palliative chemotherapy, PIPAC and PITAC have to be repeated at regular intervals (currently about 6 weeks) to be effective.

On the other side, delivery of chemotherapeutics to the peritoneum at the same time as surgery is performed, might prevent tumour recurrence, however, is often associated with wound healing problems, in particular with bowel perforations and leakage of surgical sutures.

Therefore, it was the primary object of the present invention to provide new and improved treatment measures for treating cancer or in connection with treating cancer, particularly shortly after a surgery for removing a tumour, preferably to provide new and improved delivery systems to be used in the treatment of cancer, wherein one or more, particularly preferably all, of the above-mentioned drawbacks are avoided or overcome by use of the same.

The primary object of the present invention is achieved by an anti-cancer agent delivery system comprising or consisting of
a) particles, the particles being nano- and/or microparticles, comprising or consisting of
   a.1) 50 to 98 wt.-%, preferably 60 to 97 wt.-%, particularly preferably 70 to 96 wt.-%, especially preferably 80 to 96 wt.-%, or preferably 65 to 98 wt.-%, preferably 65 to 97 wt.-%, preferably 65 to 96 wt.-%, or preferably 70 to 98 wt.-%, preferably 70 to 97 wt.-%, preferably 70 to 96 wt.-%, or preferably 72 to 98 wt.-%, preferably 72 to 97 wt.-%, preferably 72 to 96 wt.-%, or preferably 74 to 98 wt.-%, preferably 74 to 97 wt.-%, preferably 74 to 96 wt.-%, based on the total weight of the particles, of a base substance, wherein the base substance consists of
      a.1.i) one, two, three or more polymers, wherein the, one, two, three or more or all polymers is/are selected from the group consisting of poly(lactic-co-glycolic acid) (PLGA), poly(lactide), poly(lactide-co-glycolide), poly(isobutylcyanoacrylate), poly(isohexylcyanoacrylate), poly(n-butylcyanoacrylate) poly(acrylate), poly(mathacrylate), chitosan, alginate, gelatin, albumin, poly(methacrylate), poly(e-caprolactone), polylactic acid, poly(b hydroxyl butyrate), ethyl cellulose, polystyrene, poly(vinyl pyridine), poly(alkyl methacrylate) and poly(alkyl cyanoacrylate), and
      a.1.ii) optionally: one, two, three or more lipids, wherein the, one, two, three or more or all lipids are selected from the group consisting of glyceryl monostearate, glycerol mono-oleate, medium-chain triglycerides, sorbitan monostearate, sorbitan monopalmitate, sorbitan monolaurate, sorbitan monooleate, sorbitan trioleate, polyoxyethylene 20 sorbitan monostearate, polyoxyethylene 20 sorbitan mono palmitate, polyoxyethylene 20 sorbitan monolaurate, polyoxyethylene 20 sorbitan mono oleate, propylene glycol monocaprylate, propylene glycol caprylate, propylene glycol monolaurate, oleoyl macrogolglycerides, linoleoyl macrogolglycerides, polyoxyl 35 castor oil, polyoxyl 40 hydrogenated castor oil, polyoxyl 60 hydrogenated castor oil, capryl alcohol, capri alcohol, lauryl alcohol, myristyl alcohol, palmityl alcoholl, stearyl alcohol, arachidiyl alcohol, behenyl alcohol, oleyl alcohol, cetostearyl alcohol, α-linolenic acid, myristoleic acid, palmitoleic acid, oleic acid, linoleic acid,
   a.2) 0.25 to 20 wt.-%, preferably 0.5 to 15 wt.-%, particularly preferably 0.5 to 10 wt.-%, especially preferably 0.5 to 5 wt.-%, or preferably 0.25 to 7.5 wt.-%, preferably 0.5 to 6 wt.-%, preferably 0.75 to 5 wt.-%, or preferably 0.5 to 20 wt.-%, preferably 0.75 to 20 wt.-%, or preferably 0.25 to 15 wt.-%, preferably 0.75 to 15 wt.-%, or preferably 0.25 to 10 wt.-%, preferably 0.75 to 10 wt.-%, or preferably 0.5 to 7.5 wt.-%, preferably 0.75 to 7.5 wt.-%, or preferably 0.25 to 6 wt.-%, preferably 0.75 to 6 wt.-%, or preferably 0.25 to 5 wt.-%, based on the total weight of the particles, of one, two, three or more cancer chemo-therapeutic agents, and
   a.3) optionally: 0.5 to 30 wt.-%, preferably 1 to 27.5 wt.-%, particularly preferably 1.5 to 25 wt.-%, especially preferably 2 to 20 wt.-%, even further preferably 2.5 to 18 wt.-%, or preferably 0.5 to 27.5 wt.-%, preferably 1.5 to 27.5 wt.-%, preferably 2 to 27.5 wt.-%, preferably 2.5 to 27.5 wt.-%, or preferably 0.5 to 25 wt.-%, preferably 1 to 25 wt.-%, preferably 2 to 25 wt.-%, preferably 2.5 to 25 wt.-%, or preferably 0.5 to 20 wt.-%, preferably 1 to 20 wt.-%, preferably 1.5 to 20 wt.-%, preferably 2.5 to 20 wt.-%, or preferably 0.5 to 18 wt.-%, preferably 1 to 18 wt.-%, preferably 1.5 to 18 wt.-%, preferably 2 to 18 wt.-%, based on the total weight of the particles, of one, two, three or more surface coating material(s), wherein the, one, two, three or more or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin and pullulan,
   a.4) optionally: 1 to 10 wt.-%, based on the total weight of the particles of one, two, three or more further active substances, wherein the, one, two, three or more or all further active substances are selected from the group consisting of immunomodulatory drugs, antibodies, viruses, adjuvants such as vitamins and enzymes, and combinations thereof,
   a.5) 0.5 to 20 wt.-%, preferably 1 to 20 wt.-%, preferably 0.5 to 15 wt.-%, preferably 1 to 15 wt.-%, preferably 0.5 to 10 wt.-%, preferably 0.5 to 5 wt.-%, based on the total weight of the particles of one, two, three or more
      - stabilizers, preferably surfactants, particularly preferably of one, two, three or more or all surfactants are selected from the group consisting of cationic surfactants, non-ionic surfactants and/or anionic surfactants,
      and/or preferably
      - substances selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid
   and wherein the average size of the particles is in a range of from 100 to 5000 nm (i.e. 5 µm),
   wherein in case component a.1.ii) is not present, the average size of the particles is in a range of from 400 to 5000 nm,
b) optionally: one or more further pharmaceutically acceptable components,
   wherein in an in vitro assay, comprising or consisting of the following steps:
   1) providing the anti-cancer agent delivery system, wherein the amount of the anti-cancer agent(s) is pre-determined,
   2) adding the anti-cancer agent delivery system to a pre-determined amount of liquid medium which does not contain the anti-cancer agent(s) of the delivery system, and
   3) analysing the amount of the anti-cancer agent(s) released from the delivery system into the medium after a pre-determined time or after pre-determined times,
   the release of more than 5 wt.-%, preferably 4 wt.-%, 3 wt.-%, 2 wt.-%, 1 wt.-%, 0.5 wt.-%, 0.1 wt.-% of component a.2), based on the total weight of component a.2), to the medium is prevented for at least 2 hours, preferably at least 5 hours, preferably at least 8 hours preferably at least 12 hours preferably at least 24 hours preferably at least 36 hours preferably at least 48 hours preferably at least 72 hours preferably at least 90 hours preferably at least 120 hours after the addition of the anti-cancer agent delivery system or the particles in step 2),
   for use in the treatment of cancer in a human subject, wherein the treatment comprises one or more applications of the delivery system to the human subject, preferably by direct introduction into the abdomen and/or thorax or other body cavity or hollow organ. Before introduction, the delivery system can be diluted. Preferably, the term "introduction into the abdomen and/or thorax" also includes an introduction into a hollow organ located in the abdomen and/or thorax.

Preferably, the particles of the active substance delivery system comprise or consist of micro- and nanoparticles as described above, wherein both, components a.1.i) and a.1.ii) are present and the average size of the particles is in a range of from 100 to 5000 nm, preferably of from 100 to 3500 nm, particularly preferably of from 100 to 2500 nm, especially preferably of from 100 to 900 nm.

Also preferably, the particles of the active substance delivery system consist of microparticles, wherein component a.1.ii) is not present and the average size of the microparticles is in a range of from 400 to 5000 nm, preferably of from 400 to 3500 nm, particularly preferably of from 400 to 2500 nm, especially preferably of from 100 to 900 nm.

Also preferably, the particles of the active substance delivery system consist of nanoparticles, wherein component a.1.ii) is present and the average size of the nanoparticles is in a range of from 100 to <400 nm.

Also preferably, the particles of the active substance delivery system consist of nanoparticles, wherein component a.1.ii) is present and the average size of the nanoparticles is in a range of from 400 to 5000 nm, preferably of from 400 to 3500 nm, particularly preferably of from 400 to 2500 nm, especially preferably of from 400 to 900 nm.

Preferably, the term "comprising or consisting of nano- and/or microparticles" as used herein is meant to be understood such that a substantial amount or all of the present particles is classified as nano- and/or microparticles according to the definition as used hereinbelow.

Preferably, the term "consisting of microparticles" as used herein is meant to be understood such that a substantial amount or all of the present particles is classified as microparticles according to the definition as used hereinbelow.

Preferably, the term "consisting of nanoparticles" as used herein is meant to be understood such that a substantial amount or all of the present particles is classified as nanoparticles according to the definition as used hereinbelow.

The term "microparticles" as used herein includes particles with a particle size of from 400 to 5000 nm preferably of from 400 to 3500 nm, particularly preferably of from 400 to 2500 nm, especially preferably of from 100 to 900 nm.

The term "nanoparticles" as used herein includes particles with a particle size of from 100 to 400 nm.

The term "nano- and/or microparticles" as used herein thus preferably includes particles with a particle size of from 100 to 5000 nm, preferably of from 100 to 3500 nm, particularly preferably of from 100 to 2500 nm, especially preferably of from 100 to 900 nm.

Preferably, the highest release rate of anti-cancer agent(s), i.e. the highest amount of anti-cancer agent(s) released per time, e.g. per second, per minute, per hour or per day, is not observed before 2 hours, preferably 4 hours, 6 hours, or 8 hours after the addition of the anti-cancer agent delivery system or the particles in step 2).

Preferably, a release of anti-cancer agent(s) can be measured up to 7 days, 10 days, 20 days, 30 days, 40 days, 50 days, 60 days, 70 days, 80 days or 90 days after the addition of the anti-cancer agent delivery system or the particles in step 2).

The term "a release of the anti-cancer agent(s) can be measured up to 7 days" is meant to be understood such that the delivery system releases the anti-cancer agent(s) at least until 7 days after the addition of the anti-cancer agent delivery system to the liquid medium. The release may also be interrupted once or several times until the end of 7 days, however, has to be resumed once or several times until the end of 7 days. The same applies for other time points, e.g. 10 days, 20 days, 30 days, 40 days, 50 days, 60 days, 70 days, 80 days or 90 days, accordingly.

Preferably in the in vitro assay, the total delivery system or, respectively, the particles is/are enveloped with a membrane, which allows the medium and the anti-cancer agent(s), however, not the delivery system or, respectively, the particles as such to pass. The term "enveloped" as used herein describes a condition in which the delivery system or, respectively, the particles is/are completely surrounded by the membrane. The membrane may be any means suitable to allow the medium and the dissolved and/or optionally the released anti-cancer agent(s), however, not the delivery system or, respectively, the particles as such to pass. The membrane may be of any suitable material. Preferably, the membrane is a polyvinylidene fluoride (PVDF) membrane, a polyester (PE) membrane, a polyether sulfone (PES) membrane, a nylon membrane or a polycarbonate membrane or other suitable synthetic or natural membrane or barrier or biological layer. Preferably, the pore size of the membrane is in the range of from 0.001 µm to 0.1 µm.

Preferably, the enveloped delivery system or, respectively, the particles is/are then added to the liquid medium (packed in barrier or membrane as described above).

Preferably, the liquid medium is water, saline, glucose solution, phosphate buffer saline (PBS), tris buffered saline (TBS), 4-(2-hydroxyethyl)-1-piperazineethanesulfonic acid (HEPES) buffer, or any other physiological fluid or a mixture of them at suitable ratio, e.g. in certain embodiments the liquid medium is a mixture consisting of 50 vol.-% of an artificial peritoneal dialysis fluid and 50 vol.-% of saline, and thus does not contain the anti-cancer agent(s) of the delivery system. Typically, an isotonic saline solution containing 9 g of sodium chloride per liter is used as saline. An artificial peritoneal dialysis fluid may have the following composition:

| **Composition of the final solution after mixing in mmol/L** | |
|---|---|
| Anhydrous glucose (C₆H₁₂O₆) | 126 |
| Sodium (Na⁺) | 132 |
| Magnesium (Mg⁺⁺) | 1.25 |
| Calcium (Ca⁺⁺) | 0.25 |
| Chloride (Cl⁻) | 95 |
| Bicarbonate (HCO₃⁻) | 25 |
| Lactate | 15 |
| Osmolarity | 395 mOsmol/L |

Preferably in the in vitro assay, the medium is continuously stirred at 50 to 500 rpm, preferably 50 to 250 rpm. Preferably, the medium is continuously stirred at 100- 200 rpm, particularly preferably at 150 rpm.

Preferably for stirring, a magnetic stir, a paddle, a mixer, or sonicator can be used.

Preferably, the medium is stirred at a temperature in a range of from 19 to 25 °C, preferably of from 20 to 23 °C, particularly preferably at 20 °C.

Particularly preferably the assay is performed under standard conditions, i.e. at room temperature (i.e. 20-25 °C, for example 25 °C), and, preferably, at 1013 mbar.

Preferably in the in vitro assay, the amount of medium ranges between 25 and 500 ml, preferably, 50 and 450 ml, particularly preferably 75 to 400 ml, especially preferably 100 to 300 ml.

Typically, samples of e.g. 2 ml of the medium are taken 2 hours, 5 hours, 8 hours, 12 hours, 24 hours, 36 hours, 48 hours, 72 hours, 90 hours, 120 hours, and preferably also 7 days, 10 days, 15 days, 30 days, 60 days and/or 90 days after the addition of the anti-cancer agent delivery system and preferably, the collected medium is replaced by the same amount (e.g. 2 ml) of the same medium (which was freshly prepared). Other time points or only several of these time points may also be suitable.

Subsequently, the collected samples are analysed for their content of the anti-cancer agent(s) by means of HPLC, however other suitable techniques are also known to a skilled person and may also be used.

As the amount of the anti-cancer agent(s) is pre-determined, as is the amount of the liquid medium, the amount of the anti-cancer agent(s) measured in the sample taken (the volume of which is known) can be translated to the amount of the anti-cancer agent(s) in the liquid medium and thus the released amount (e.g. in wt.-%) of the anti-cancer agent(s) from the delivery system or, respectively, the particles. A similar release into e.g. body fluids of the treated subject such as the blood plasma can be expected. Said similarity may however depend on the particular liquid medium selected for the in vitro assay.

According to one aspect of the present invention, the delivery system is introduced into a subject, particularly into the abdomen and/or thorax of the subject. The release of the anti-cancer agent may in some cases also be measured by obtaining biological samples of the subject before, during and/or after treatment, analysing the concentration of the anti-cancer agent in said biological samples and optionally comparing said concentrations with each other. Such biological samples may e.g. be tissue samples or samples of body fluids such as blood samples. Preferably, the anti-cancer agent is not present in the subject before the treatment, i.e. before the (first) introduction of the delivery system.

Preferably the subject can be human or animal, preferably a human suffering from cancer, such as a cancer of any body cavity or a hollow organ, preferably a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumour of embryonal origin.

The treatment as described herein can comprise or consist of a parenteral administration of the delivery system, preferably an intraperitoneal and/or intrathoracic administration of the delivery system and/or preferably an administration of the delivery system to any other body cavity or hollow organ.

The treatment as described herein preferably comprises an administration of the delivery system before, during or after surgery, preferably by systemic application, preferably an oral or intravenous application, or by local application, preferably an intraperitoneal application.

The treatment of cancer in a human subject can be any kind of treatment of cancer. Particularly, the treatment is directed to cancer in a body cavity or a hollow organ. Preferably, the treatment is directed to a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

The treatment as described herein can be directed to reducing tumour size and the amount of tumour sites. Tumour sites includes the primary tumour as well as metastases and further tumour reproductions. The treatment can also be directed to prevent or delay cancer recurrence after tumour removal or partial tumour removal, preferably after a surgical tumour removal.

The anti-cancer agent delivery system may be used in several treatments, such as local or subcutaneous or muscular treatments. An example of such a treatment is PIPAC as described above.

It is of particular advantage, that due to the delayed release, the anti-cancer agents in the anti-cancer agent delivery system do not (significantly) impair wound healing. The term "delayed" release preferably describes a release which does not start immediately at the time point of administration; particularly preferably, a substantial release does not start immediately at the time point of administration as is described herein.

It is also of particular advantage, that due to the sustained release, the presence of anti-cancer agents in the subject to which the delivery system according to the invention is applied can be significantly prolonged, which in turn reduces the number of additional medical interventions such as further PIPAC or PITAC or other surgical procedures including anaesthesia. Preferably, the term "sustained" release describes a release which occurs or, respectively, can be measured over several weeks, such as described herein. The term "sustained" release preferably further includes a release which may vary in its strength and may also be interrupted, wherein the term "interrupted" necessarily includes that the release is resumed.

It is preferred that the treatment comprises one, two, three or more administration(s) of the delivery system, particularly preferably one administration of the delivery system.

Preferably, the or one, two, three or all administration(s) of the delivery system is/are performed with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

Preferably, the, one, two or more or all anti-cancer agents used within the scope of the present invention is/are selected from the group consisting of anti-cancer agents used against a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

For example, such anti-cancer agents may be selected from the group consisting of cisplatin, doxorubicin, paclitaxel and oxaliplatin and a combination thereof.

Preferably, the anti-cancer agent(s) is/are dissolved in a solvent. In some cases, the solvent can also be added in a later step during a process of manufacture of the delivery system or the solvent may be partially or completely removed during such a process, as it may be the case for some solid forms of the delivery system.

The solvent(s) for dissolving the anti-cancer agent(s) is/are preferably solvents that are pharmaceutically acceptable. Preferably, the solvents comprise or consist of one or more substances selected from the group consisting of acetone ethyl acetate, chlorinated solvents, DMSO, methylated solvents, tetrahydrofuran, halogenated hydrocarbons such as chloroform and dichloromethane, dioxanes, acetonitrile, polyethylene glycol, such as polyethylene glycol 400; N-methylpyrrolidone; ethanol; dimethylacetamide; propylene glycol and combinations thereof.

Preferably, the solvent is not considered when calculating the weight percentages of the anti-cancer agent(s).

Preferably, component a2) of the particles includes a combination of polymers, preferably all polymers of the combination of polymers are selected from the group consisting of poly(lactic-co-glycolic acid) (PLGA), poly(lactide), poly(lactide-co-glycolide), poly(isobutylcyanoacrylate), poly(isohexylcyanoacrylate), poly(n-butylcyanoacrylate) poly(acrylate), poly(mathacrylate), chitosan, alginate, gelatin, albumin, poly(methacrylate), poly(e-caprolactone), polylactic acid, poly(b hydroxyl butyrate), ethyl cellulose, polystyrene, poly(vinyl pyridine), poly(alkyl methacrylate) and poly(alkyl cyanoacrylate).

According to the present invention, the anti-cancer agent(s) may be dissolved in one or more solvents, i.e. in a single solvent or a solvent mixture. Furthermore, in case of more than one agent, the agents can either be dissolved in the same solvent / solvent mixture or can be dissolved in different solvents / solvent mixtures.

The amount of the or, respectively, each anti-cancer agent is preferably contained in a range of from 120 mg to 2500 mg, preferably of from 150 mg to 2000 mg, particularly preferably of from 150 mg to 1500 mg, especially preferably of from 150 mg to 800 mg in the delivery system or, respectively, the particles, preferably this indication refers to the total amount administered during one application, preferably as a powder or suspended or redispersed in physiological solutions like saline. The delivery system may be diluted in saline or any other physiological solution, which is pharmaceutically acceptable, before administration.

Additionally or alternatively, the amount of the or, respectively, each anti-cancer agent is preferably contained in a range of from 30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface in the delivery system or, respectively, the particles, wherein this indication refers to the total amount administered during one application, preferably as a powder or suspended or redispersed in physiological solutions like saline. The amounts of the anti-cancer agent may, however, vary with the respective anti-cancer agent(s) and/or type of anti-cancer agent(s). Thus, the amount of the or, respectively, each anti-cancer agent as described may e.g. also be contained in a range of from 50 mg/m² body surface to 70 mg/m² body surface.

The term "body surface" as used herein refers to the body surface of the human subject receiving the delivery system according to the invention or, respectively, the particles according to the invention. The body surface can be calculated from the size of the human subject and its body weight and is well known to a person skilled in the art.

Preferably, the one or more further pharmaceutically acceptable component(s) is/are selected from the group consisting of carriers, polymers, wetting agents, emulsifiers, antioxidants, pH influencing agents, disintegrants, recrystallization agents, fluxing agents, preservatives, solvents, salts, fillers, binders, foamers, defoamers, lubricants, adsorbents, substances for adjusting the osmotic pressure and buffers.

The stabilizer(s), as present, advantageously stabilize the electro-stability of the particles and avoid aggregation, avoid polymer degradation or crystallization of lipids and further particle growth, thereby providing long term stability. At optimum concentration they facilitate drug release from the nanoparticle/microparticle core. Thus the particles according to the invention comprise as component a.5) one, two, three or more stabilizers, preferably surfactants, particularly preferably of one, two, three, or more or all surfactants selected from the group consisting of cationic surfactants, non-ionic surfactants and/or anionic surfactants,
preferably one, two, three or more substances selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid,
wherein for the calculation of the weight ratio of component a.5), the sum of the weights of the stabilizers, preferably surfactants, and the substances selected from the group specified above is considered.

It is particularly preferred if the delivery system as described herein or the particles as described herein can be administered with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

The delivery system according to the invention can for example be present in form of a powder (e.g. micron, submicron or nanosized), microspheres, pellets, granules, an aerosol, i.e. as liquid or solid particle(s) in a gas or mixture of gases, a suspension or an emulsion. If present in liquid or in solid form, such as powder, pellets or granules, the delivery system can be mixed with a gas or a mixture of gases to produce an aerosol. Alternatively, if present in solid form, the delivery system can be dissolved or dispersed in a suitable solvent / solvent or diluent fluid or in suitable physiological fluid mixture before application to the subject in need thereof.

Furthermore, the present invention relates to a method for producing a delivery system, according to the invention, or, respectively, particles, the particles being nano- and/or microparticles according to the invention, comprising or consisting of the steps
i) providing components a.1.i) and optionally a.1.ii) and dissolving these in a solvent,
ii) providing component a.2) and dissolving it in a solvent,
   ii.a) optionally providing component a.4) and dissolving it in a solvent,
   ii.b) providing component a.5)
iii) mixing the solutions obtained in step i) and ii) and ii.a) if present and ii.b),
iv) providing a polyvinyl alcohol (PVA) solution, preferably a solution containing 0.1 to 5 wt.-%, particularly preferably 0.2 to 3 wt.-% of PVA, based on the total amount of the solution,
v) adding the mixture obtained in step iii) to the solution obtained in step iv) to produce particles,
vi) optionally: evaporating the solvent, one of or the solvents used in step i) and ii), and
vii) purifying the particles and subsequently dispersing the particles in a dispersant, preferably in a pharmaceutically acceptable solvent,
viii) providing one, two, three or more surface coating material(s) and coating the particles therewith,
ix) optionally: providing and adding one or more further pharmaceutically acceptable components to the particles
   wherein in step v) the amounts of the mixture and of the solution are selected such that a ratio of a least 1 ml of solution per 5 mg of the summed weight of component a.1.i) and the anti-cancer agent(s) is achieved, and
   wherein the addition in step v) is performed by injection, preferably constant injection, preferably at a rate in a range of from 0.5 to 5 ml/min, preferably in a range of from 0.5 to 3,0 ml/min, particularly preferably at a rate in a range of from 0.4 to 2,5 ml/min
   and preferably wherein the weight ratio of PLGA and the solvent used in step i) is in a range of from 2.5 to 40, preferably of from 3 to 30.

The term "providing a substance and dissolving the substance in a solvent", as used herein, e.g. in steps i) or ii) of the method above, are meant to be understood such that the respective substance may be provided first and subsequently be dissolved in a respective solvent, but also includes the case that a substance already dissolved in a solvent is provided.

The term "solvent" as used herein can be a single substance but also a mixture of substances. Thus, "solvent" may also encompass several different substances, e.g. several different single solvents.

Preferably, the solvent used in step i) and/or the solvent used in step ii) of the method as described herein comprises or consists of one or more substances selected from the group consisting of acetone ethyl acetate, chlorinated solvents, DMSO, methylated solvents, tetrahydrofuran, halogenated hydrocarbons such as chloroform and dichloromethane, dioxanes, acetonitrile, polyethylene glycol, such as polyethylene glycol 400; N-methylpyrrolidone; ethanol; dimethylacetamide; propylene glycol and combinations thereof.

It is further preferred that additionally or alternatively, the solvent used in step iv) for providing a PVA solution comprises or consists of one or more substances selected from the group consisting of water, ethanol and saline.

Additionally, or alternatively, the dispersant used in step vii) comprises or consists of one or more substances selected from the group consisting of saline, glucose, surfactants, pH modifiers and water.

It is particularly preferred if the, one or all anti-cancer agent(s) of component a1) of the delivery system according to the invention or of particles as described herein is/are (a) anti-cancer agent(s), preferably selected from the group consisting of anti-cancer agents used against a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, particularly preferably selected from the group consisting of paclitaxel, cisplatin, oxaliplatin and doxorubicin.

Furthermore and preferably, the amount of the or, respectively, each anti-cancer agent is preferably contained in a range of from 120 mg to 2500 mg, preferably of from 150 mg to 2000 mg, particularly preferably of from 150 mg to 1500 mg, especially preferably of from 150 mg to 800 mg in the delivery system or, respectively, the particles, preferably this indication refers to the total amount administered during one application, preferably as a powder or suspended or redispersed in physiological solutions like saline. The delivery system may be diluted in saline or any other physiological solution, which is pharmaceutically acceptable, before administration.

Additionally or alternatively, the amount of the or, respectively, each anti-cancer agent is preferably contained in a range of from 30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface in the delivery system or, respectively, the particles, wherein this indication refers to the total amount administered during one application, preferably as a powder or suspended or redispersed in physiological solutions like saline. The amounts of the anti-cancer agent may, however, vary with the respective anti-cancer agent(s) and/or type of anti-cancer agent(s). Thus, the amount of the or, respectively, each anti-cancer agent as described, may e.g. also be contained in a range of from 50 mg/m² body surface to 70 mg/m² body surface.

Further preferred is if the, one, two, three or more or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin and pullulan.

The coating is preferably performed by covalent attachment or by crosslinking by adding a surface modified agent into one of the phases. It is also possible to use a modified polymer as a starting material.

Additionally, or alternatively, the coating is preferably performed by a one pot synthesis adding a hydrophilic compound in the polar medium. Using such an approach and a polyelectrolyte of the respective charge the overall surface potential of the particles can be adapted. This might be of use for improved interaction with cell membranes, improved uptake but also for modified deposition behaviour depending on the setup.

As another preferred approach the consecutive coating might be used additionally or alternatively. Preparation of loaded particles and then the addition to the aqueous polymeric solution for adsorption and surface coating may also be an option.

Further preferred is if additionally, or alternatively, the total amount of the surface coating material(s) is in a range of from 1 to 20 wt.-%, preferably in a range of from 3 to 15 wt.-%, particularly preferably in a range of from 5 to 10 wt.-%, based on the total weight of the particles.

Preferably, the one or more further pharmaceutically acceptable component(s) is/are selected from the group consisting of carriers, polymers, wetting agents, emulsifiers, antioxidants, pH influencing agents, disintegrants, recrystallization agents, fluxing agents, preservatives, solvents, salts, fillers, binders, foamers, defoamers, lubricants, adsorbents, substances for adjusting the osmotic pressure and buffers.

It is also preferred if the addition of the mixture, obtained in step iii), in step v) of the described method is performed by injection, i.e. by slowly adding the mixture to the polyvinyl alcohol (PVA) solution with a suitable way, preferably by using a syringe pump (equipped with a syringe and a cannula). Preferably, the injection is a constant injection, especially preferably at a rate in a range of from 0.5 to 5 ml/min, preferably in a range of from 0.5 to 3,0 ml/min, particularly preferably at a rate in a range of from 0.4 to 2,5 ml/min to avoid aggregate formation. The optimum flow is the key parameter to obtain uniform particle size dispersion. The quality of the particles in terms of average size but also the size distribution can thus be improved by using such a technique. Depending on the rate of the constant injection, particle sizes of different values can be obtained. In this case, a low injection rate will create particles of smaller size, whereas a high injection rate will create particles of larger size or may be aggregation, whereas preferably a processing step like homogenization is also performed either before, after or subsequently. The skilled person also knows that the particular recipe, i.e. the ingredients and their ratios, will influence the particular particle size of the prepared particles.

The term constant injection as used herein describes a constant injection rate after an initial phase and before an end phase. In the initial phase and in the and end phase, the injection rate is typically lower than the intended constant injection rate and is increased to achieve the intended constant injection rate or, respectively, decreases as the material to be injected fades and may no longer uphold the intended constant injection rate. In the initial phase, while the constant injection rate is adjusted, the injection rate may also exceed the intended constant injection rate. It is preferred that 75 wt.-% of the material to be injected, preferably 80 wt.-%, particularly preferably 90 wt.-% or 95 wt.-%, is injected by means of constant injection as described herein.

Typically, the particle size of the particles is measured by dynamic light scattering techniques and electron microscopy or scanning probe microscopy. The size can also be determined by fractionation with disc centrifugation and flow techniques. Further methods are known to a person skilled in the art and may also be applied. It is preferred that the average particle size as described herein is defined by applying such methods. Particularly, the average particle size as described herein is defined by applying dynamic light scattering techniques, electron microscopy, scanning probe microscopy, fractionation with disc centrifugation, flow techniques and/or other methods known to a person skilled in the art. Preferably, the average particle size describes the average of the particle sizes of a plurality of particles determined as described above. Furthermore, when measuring the (average) particle size, preferably the diameter of the respective particle(s) is considered.

Also described herein is an anti-cancer agent delivery system, as described herein, *per se,* i.e. independent of its use. The anti-cancer agent delivery system comprises or consists of
a) particles, the particles being nano- and/or microparticles, comprising or consisting of
   a.1) 50 to 98 wt.-%, preferably 60 to 97 wt.-%, particularly preferably 70 to 96 wt.-%, especially preferably 80 to 96 wt.-%, or preferably 65 to 98 wt.-%, preferably 65 to 97 wt.-%, preferably 65 to 96 wt.-%, or preferably 70 to 98 wt.-%, preferably 70 to 97 wt.-%, prefer-ably 70 to 96 wt.-%, or preferably 72 to 98 wt.-%, preferably 72 to 97 wt.-%, preferably 72 to 96 wt.-%, or preferably 74 to 98 wt.-%, preferably 74 to 97 wt.-%, preferably 74 to 96 wt.-%, based on the total weight of the particles, of a base substance, wherein the base substance consists of
      a.1.i) one, two, three or more polymers, wherein the, one, two, three or more or all polymers is/are selected from the group consisting of poly(lactic-co-glycolic acid) (PLGA), poly(lactide), poly(lactide-co-glycolide), poly(isobutylcyanoacrylate), poly(isohexylcy-anoacrylate), poly(n-butylcyanoacrylate) poly(acrylate), poly(mathacrylate), chitosan, alginate, gelatin, albumin, poly(methacrylate), poly(e-caprolactone), polylactic acid, poly(b hydroxyl butyrate), ethyl cellulose, polystyrene, poly(vinyl pyridine), poly(alkyl methacrylate) and poly(alkyl cyanoacrylate), and
      a.1.ii) optionally: one, two, three or more lipids, wherein the, one, two, three or more or all lipids are selected from the group consisting of glyceryl monostearate, glycerol mono-oleate, medium-chain triglycerides, sorbitan monostearate, sorbitan monopalmitate, sorbitan monolaurate, sorbitan monooleate, sorbitan trioleate, polyoxyethylene 20 sorbitan monostearate, polyoxyethylene 20 sorbitan mono palmitate, polyoxyethylene 20 sorbitan monolaurate, polyoxyethylene 20 sorbitan mono oleate, propylene glycol monocaprylate, propylene glycol caprylate, propylene glycol monolaurate, oleoyl macrogolglycerides, linoleoyl macrogolglycerides, polyoxyl 35 castor oil, polyoxyl 40 hydrogenated castor oil, polyoxyl 60 hydrogenated castor oil, capryl alcohol, capri alcohol, lauryl alcohol, myristyl alcohol, palmityl alcoholl, stearyl alcohol, arachidiyl alcohol, behenyl alcohol, oleyl alcohol, cetostearyl alcohol, α-linolenic acid, myristoleic acid, palmitoleic acid, oleic acid, linoleic acid,
   a.2) 0.25 to 20 wt.-%, preferably 0.5 to 15 wt.-%, particularly preferably 0.5 to 10 wt.-%, especially preferably 0.5 to 5 wt.-%, or preferably 0.25 to 7.5 wt.-%, preferably 0.5 to 6 wt.-%, preferably 0.75 to 5 wt.-%, or preferably 0.5 to 20 wt.-%, preferably 0.75 to 20 wt.-%, or preferably 0.25 to 15 wt.-%, preferably 0.75 to 15 wt.-%, or preferably 0.25 to 10 wt.-%, preferably 0.75 to 10 wt.-%, or preferably 0.5 to 7.5 wt.-%, preferably 0.75 to 7.5 wt.-%, or preferably 0.25 to 6 wt.-%, preferably 0.75 to 6 wt.-%, or preferably 0.25 to 5 wt.-%, based on the total weight of the particles, of one, two, three or more cancer chemo-therapeutic agents, and
   a.3) optionally: 0.5 to 30 wt.-%, preferably 1 to 27.5 wt.-%, particularly preferably 1.5 to 25 wt.-%, especially preferably 2 to 20 wt.-%, even further preferably 2.5 to 18 wt.-%, or preferably 0.5 to 27.5 wt.-%, preferably 1.5 to 27.5 wt.-%, preferably 2 to 27.5 wt.-%, preferably 2.5 to 27.5 wt.-%, or preferably 0.5 to 25 wt.-%, preferably 1 to 25 wt.-%, preferably 2 to 25 wt.-%, preferably 2.5 to 25 wt.-%, or preferably 0.5 to 20 wt.-%, preferably 1 to 20 wt.-%, preferably 1.5 to 20 wt.-%, preferably 2.5 to 20 wt.-%, or preferably 0.5 to 18 wt.-%, preferably 1 to 18 wt.-%, preferably 1.5 to 18 wt.-%, preferably 2 to 18 wt.-%, based on the total weight of the particles, of one, two, three or more surface coating material(s), wherein the, one, two, three or more or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin and pullulan,
   a.4) optionally: 1 to 10 wt.-%, based on the total weight of the particles of one, two, three or more further active substances, wherein the, one, two, three or more or all further active substances are selected from the group consisting of immunomodulatory drugs, antibodies, viruses, adjuvants such as vitamins and enzymes, and combinations thereof,
   a.5) 0.5 to 20 wt.-%, preferably 1 to 20 wt.-%, preferably 0.5 to 15 wt.-%, preferably 1 to 15 wt.-%, preferably 0.5 to 10 wt.-%, preferably 0.5 to 5 wt.-%, based on the total weight of the particles of one, two, three or more
      - stabilizers, preferably surfactants, particularly preferably of one, two, three or more or all surfactants are selected from the group consisting of cationic surfactants, non-ionic surfactants and/or anionic surfactants,
      and/or preferably
      - substances selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid
      and wherein the average size of the particles is in a range of from 100 to 5000 nm,
      wherein in case component a.1.ii) is not present, the average size of the particles is in a range of from 400 to 5000 nm,
b) optionally: one or more further pharmaceutically acceptable components,
   wherein in an in vitro assay, comprising or consisting of the following steps:
   1) providing the anti-cancer agent delivery system, wherein the amount of the anti-cancer agent(s) is pre-determined,
   2) adding the anti-cancer agent delivery system to a pre-determined amount of liquid medium which does not contain the anti-cancer agent(s) of the delivery system, and
   3) analysing the amount of the anti-cancer agent(s) released from the delivery system into the medium after a pre-determined time or after pre-determined times,
   the release of more than 5 wt.-%, preferably 4 wt.-%, 3 wt.-%, 2 wt.-%, 1 wt.-%, 0.5 wt.-%, 0.1 wt.-% of component a.2), based on the total weight of component a.2), to the medium is prevented for at least 2 hours, preferably at least 5 hours, preferably at least 8 hours preferably at least 12 hours preferably at least 24 hours preferably at least 36 hours preferably at least 48 hours preferably at least 72 hours preferably at least 90 hours preferably at least 120 hours after the addition of the anti-cancer agent delivery system or the particles in step 2).

What is said herein with regard to the delivery system for use, including what was said with regard to the in vitro assay, also applies for preferred embodiments of the delivery system *per se.*

The present invention also relates to a mixture for use in the treatment of cancer, such as any cancer of a body cavity or a hollow organ. Preferably the cancer type is selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

The mixture according to the invention can for example be present in form of a powder, pellets, granules, an aerosol, i.e. as liquid or solid particle(s) in a gas or mixture of gases, a suspension or an emulsion. Preferably the mixture is present in a form which can be administered to a human subject. Further preferably, a pharmaceutically acceptable solvent can be added to the mixture to ease the application to the subject, preferably human subject.

Preferably, the mixture is a mixture for use as described for the delivery system according to the invention.

It is also preferred that the mixture comprises a delivery system or particles according to the invention. Further preferably, the mixture comprises two, three, four, five or more different delivery systems or, respectively, particles according to the invention. The delivery systems or, respectively, particles can for example differ in the respective anti-cancer agent / the mixture of anti-cancer agents and/or in the further composition of the delivery systems or, respectively, particles.

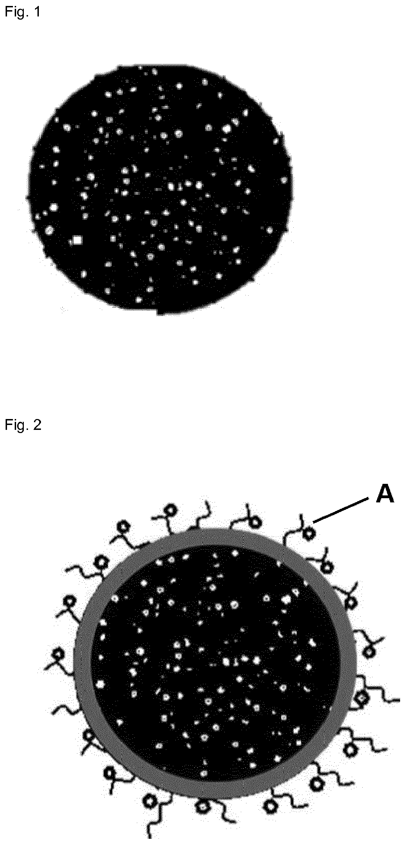

Figure 1 describes an exemplary particle according to the invention, wherein component a.1.ii) is not present.
Figure 2 describes an exemplary particle with surface coating (A) according to the invention, wherein component a.1.ii) is not present.

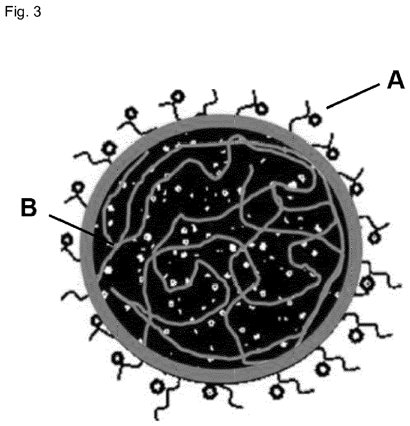

Figure 3 describes an exemplary particle with surface coating (A) according to the invention, wherein component a.1.ii) is present (B, grey lines).

Preferred embodiments and further aspects of the present invention also emerge from the attached patent claims and the following examples, wherein the present invention is not limited to these examples.

### Examples:

### Example 1:

| Ingredient | Amount [mg] | | | | | | | | | |
|---|---|---|---|---|---|---|---|---|---|---|
| | A | B | C | D | E | F | G | H | I | J |
| PLGA | 15 | - | - | - | 1 | 2 | 3 | 4 | 5 | 6 |
| PCL | - | 50 | 30 | 40 | - | - | - | - | - | - |
| Triglyceride | - | - | - | - | 10 | 20 | 30 | 50 | 50 | 50 |
| Paclitaxel | 0.75 | 0.25 | 0.50 | 0.75 | 0.25 | 0.50 | 0.75 | 0.75 | 0.75 | 0.75 |
| Acetone | 3.5 | - | - | - | 1.5 | 2.5 | 3.5 | - | - | - |
| Polyvinylalcohol (PVA) | 0.8 | 1 | 1.2 | 1.2 | 0.5 | 1 | 1.2 | 1.2 | 1.2 | 1.2 |
| Dichloromethane | - | 1.5 | 2.5 | 3.5 | - | - | - | 1.5 | 2.5 | 3.5 |
| Aceton itri le | 0.5 | 0.15 | 0.30 | 0.50 | 0.15 | 0.30 | 0.50 | 0.15 | 0.30 | 0.50 |
| Chitosan | 2.7 | 1.5 | 2.1 | 2.7 | - | - | - | - | - | - |
| Sodium alginate | - | - | - | - | 1.5 | 2.1 | 2.7 | 1.5 | 2.1 | 2.7 |
| Polysorbate 80 | 0.2 | 0.6 | 0.3 | 0.3 | 0.2 | 0.2 | 0.3 | 0.3 | 0.3 | 0.3 |
| Water | q.s. | | | | | | | | | |

PLGA or PCL (poly(e-caprolactone)) was provided and dissolved in acetone or dichloromethane. For some examples, also Triglyceride was provided. Additionally, paclitaxel was provided separately and dissolved in acetonitrile. All solutions were mixed together and added to an aqueous phase containing Polysorbate 80 as indicated.

10 ml of a 2 % polyvinyl alcohol (PVA) solution were prepared, wherein the polyvinyl alcohol was dissolved in water.

The mixture obtained above was added to the PVA solution by injection with a syringe pump with a constant rate as indicated below. Upon addition, particles, i.e. nano- and/or microparticles, were produced.

| | A | B | C | D | E | F | G | H | I | J |
|---|---|---|---|---|---|---|---|---|---|---|
| Injection rate [ml/min] | 0.8 | 3 | 1.5 | 1.8 | 0.5 | 0.7 | 0.9 | 0.95 | 1 | 1.7 |

Furthermore, the particles were coated with chitosan or sodium alginate. The particles were further purified by centrifugation at 15.000 x g or other suitable technique like size exclusion chromatography at 20 °C. The supernatant was removed and the pelleted particles were redispersed in 5-10 ml of water and 0.05-0.25 g of mannitol. In another approach, the pelleted particles were redispersed in 5 ml of water and were lyophilised using 0.05-0.25 g of cryoprotectant.

The particles had an average size (diameter) as indicated below:

| | A | B | C | D | E | F | G | H | I | J |
|---|---|---|---|---|---|---|---|---|---|---|
| Average particle size [nm] | 500 | 3500 | 950 | 1200 | 278 | 320 | 600 | 758 | 870 | 980 |

### Application example 1: Release profile

The compositions as in Example 1 were tested as follows:
5 mL of the composition were added to a PVDF membrane with a pore size of approx. 0.01 µm, which allows the medium and the anti-cancer agent(s), however, not the delivery system or, respectively, the particles as such to pass. Therefore, the delivery systems or, respectively, the particles were enveloped. The enveloped delivery systems or, respectively, particles are each added to a separate medium mixture of 200 ml of artificial peritoneal dialysis fluid, which is warmed to 37 °C and continuously stirred at 100 rpm.

2 hours, 5 hours, 8 hours, 12 hours, 24 hours, 36 hours, 48 hours, 72 hours, 90 hours, 120 hours, and preferably also 7 days, 10 days, 15 days, 30 days, 60 days and/or 90 days after the addition of the enveloped delivery systems, a sample of 2 ml of the medium is taken and replaced with fresh medium.

The content of Paclitaxel in the samples was analysed by applying HPLC. No content could be measured in the medium until 2 hours, thus, there was no release. However, at a later time, a release could be measured. A release until numerous days was measured.

## Claims

1. Active substance delivery system comprising or consisting of
a) particles, the particles being nano- and/or microparticles, comprising or consisting of
a.1) 50 to 98 wt.-%, preferably 60 to 97 wt.-%, particularly preferably 70 to 96 wt.-%, especially preferably 80 to 96 wt.-%, based on the total weight of the particles, of a base substance, wherein the base substance consists of
a.1.i) one, two, three or more polymers, wherein the, one, two, three or more or all polymers is/are selected from the group consisting of poly(lactic-co-glycolic acid) (PLGA), poly(lactide), poly(lactide-co-glycolide), poly(isobutylcyanoacrylate), poly(isohexylcyanoacrylate), poly(n-butylcyanoacrylate) poly(acrylate), poly(methacrylate), chitosan, alginate, gelatin, albumin, poly(methacrylate), poly(e-caprolactone), polylactic acid, poly(b hydroxyl butyrate), ethyl cellulose, polystyrene, poly(vinyl pyridine), poly(alkyl methacrylate) and poly(alkyl cyanoacrylate), and
a.1.ii) optionally: one, two, three or more lipids, wherein the, one, two, three or more or all lipids are selected from the group consisting of glyceryl monostearate, glycerol mono-oleate, medium-chain triglycerides, sorbitan monostearate, sorbitan monopalmitate, sorbitan monolaurate, sorbitan monooleate, sorbitan trioleate, polyoxyethylene 20 sorbitan monostearate, polyoxyethylene 20 sorbitan mono palmitate, polyoxyethylene 20 sorbitan monolaurate, polyoxyethylene 20 sorbitan mono oleate, propylene glycol monocaprylate, propylene glycol caprylate, propylene glycol monolaurate, oleoyl macrogolglycerides, linoleoyl macrogolglycerides, polyoxyl 35 castor oil, polyoxyl 40 hydrogenated castor oil, polyoxyl 60 hydrogenated castor oil, capryl alcohol, capri alcohol, lauryl alcohol, myristyl alcohol, palmityl alcoholl, stearyl alcohol, arachidiyl alcohol, behenyl alcohol, oleyl alcohol, cetostearyl alcohol, α-linolenic acid, myristoleic acid, palmitoleic acid, oleic acid, linoleic acid,
a.2) 0.25 to 20 wt.-%, preferably 0.5 to 15 wt.-%, particularly preferably 0.5 to 10 wt.-%, especially preferably 0.5 to 5 wt.-% based on the total weight of the particles, of one, two, three or more cancer anti-cancer agents, and
a.3) optionally: 0.5 to 30 wt.-%, preferably 1 to 27.5 wt.-%, particularly preferably 1.5 to 25 wt.-%, especially preferably 2 to 20 wt.-%, even further preferably 2.5 to 18 wt.-%, based on the total weight of the particles, of one, two, three or more surface coating material(s), wherein the, one, two, three, or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin and pullulan,
a.4) optionally: 1 to 10 wt.-%, based on the total weight of the particles of one, two, three or more further active substances, wherein the, one, two, three or more or all further active substances are selected from the group consisting of immunomodulatory drugs, antibodies, viruses, adjuvants such as vitamins and enzymes, and combinations thereof,
a.5) 0.5 to 20 wt.-%, preferably 1 to 20 wt.-%, preferably 0.5 to 15 wt.-%, preferably 1 to 15 wt.-%, preferably 0.5 to 10 wt.-%, preferably 0.5 to 5 wt.-%, based on the total weight of the particles of one, two, three or more stabilizers, preferably surfactants, particularly preferably of one, two, three or more or all surfactants selected from the group consisting of cationic surfactants, non-ionic surfactants and/or anionic surfactants,
preferably one, two, three or more substances selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid and wherein the average size of the particles is in a range of from 100 to 5000 nm,
wherein in case component a.1.ii) is not present, the average size of the particles is in a range of from 400 to 5000 nm,
b) optionally: one or more further pharmaceutically acceptable components,
wherein in an in vitro assay, comprising or consisting of the following steps:
1) providing the anti-cancer agent delivery system, wherein the amount of the anti-cancer agent(s) is pre-determined,
2) adding the anti-cancer agent delivery system to a pre-determined amount of liquid medium which does not contain the anti-cancer agent(s) of the delivery system, and
3) analysing the amount of the anti-cancer agent(s) released from the delivery system into the medium after a pre-determined time or after pre-determined times,
the release of more than 5 wt.-%, preferably 4 wt.-%, 3 wt.-%, 2 wt.-%, 1 wt.-%, 0.5 wt.-%, 0.1 wt.-% of component a.2), based on the total weight of component a.2), to the medium is prevented for at least 2 hours, preferably at least 5 hours, preferably at least 8 hours preferably at least 12 hours preferably at least 24 hours preferably at least 36 hours preferably at least 48 hours preferably at least 72 hours preferably at least 90 hours preferably at least 120 hours after the addition of the anti-cancer agent delivery system or the particles in step 2),
for use in the treatment of cancer in a human subject, wherein the treatment comprises one or more applications of the delivery system to the human subject, preferably by direct introduction into the abdomen and/or thorax.

2. Delivery system for use according to claim 1, wherein one, the, two or more or all anti-cancer agents is/are selected from the group consisting of anti-cancer agents used against a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, preferably selected from the group consisting of cisplatin, doxorubicin, paclitaxel and oxaliplatin.

3. Delivery system for use according to claim 1 or 2, wherein in the in vitro assay, the total delivery system is enveloped with a membrane, when added to the liquid medium, which allows the medium and the anti-cancer agent(s), however, not the delivery system as such to pass,
and/or wherein the enveloped delivery system is added to the medium, which is continuously stirred at 50 to 500 rpm, preferably 50 to 250 rpm, preferably 100 to 200 rpm,
and/or wherein samples of 2 ml of the medium are taken 2 hours, 5 hours, 8 hours, 12 hours, 24 hours, 36 hours, 48 hours, 72 hours, 90 hours, 120 hours, and preferably also 7 days, 10 days, 15 days, 30 days, 60 days and/or 90 days after the addition of the anti-cancer agent delivery system and the collected medium is replaced by the same amount of the same medium and
and/or wherein the collected samples are analysed for their content of the anti-cancer agent(s) by means of HPLC.

4. Delivery system for use according to any one of the previous claims, wherein in the in vitro assay, the highest release rate of anti-cancer agent(s) is not observed before 2 hours, preferably 4 hours, 6 hours, or 8 hours after the addition of the anti-cancer agent delivery system or the particles in step 2)
and/or
wherein a release of anti-cancer agent(s) can be measured after 10 days, 20 days, 30 days, 40 days, 50 days, 60 days, 70 days, 80 days or 90 days after the addition of the anti-cancer agent delivery system or the particles in step 2).

5. Delivery system for use according to any one of the preceding claims, wherein in the treatment, the total amount of anti-cancer agent(s) administered during one application is in a range of from
120 mg to 2500 mg, preferably of from 150 mg to 2000 mg, particularly preferably of from 150 mg to 1500 mg, especially preferably of from 150 mg to 800 mg
and/or
30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface of the treated human subject.

6. Delivery system for use according to any one of the preceding claims, wherein the treatment is directed to a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

7. Delivery system for use according to any one of the preceding claims, wherein the or one, two, three or all administration(s) of the delivery system is/are performed with an assisting tool selected from the group consisting of microneedles, spray devices, angioinjectors, or any combination thereof, preferably with a nebulizer, spraying gun or spray catheter.

8. Mixture for use in the treatment of cancer, preferably a cancer type selected from the group consisting of gastrointestinal cancer, particularly gastric cancer, colorectal cancer, hepatobiliary or pancreatic cancer, appendix cancer, oesophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, comprising a delivery system as defined in any one of claims 1 to 7.

9. Mixture for use according to claim 8, comprising two, three, four, five or more different delivery systems as defined in claims 1 to 7,
wherein the delivery systems differ in the respective anti-cancer agent / the mixture of anti-cancer agents and/or in the further composition of the particles.

10. Delivery system for use according to any one of claims 1 to 7 or mixture for use according to claim 8 or 9, wherein the treatment is directed to prevent or delay cancer recurrence after a surgical tumour removal.

11. Method for producing an anti-cancer agent delivery system according to one of the preceding claims, or particles as defined in any one of the preceding claims, comprising or consisting of the following steps
i) providing components a.1.i) and optionally a.1.ii) and dissolving these in a solvent,
ii) providing component a.2) and dissolving it in a solvent,
ii.a) optionally providing component a.4) and dissolving it in a solvent,
ii.b) providing component a.5),
iii) mixing the solutions obtained in step i) and ii) and ii.a) if present and ii.b),
iv) providing a polyvinyl alcohol (PVA) solution, preferably a solution containing 0.1 to 5 wt.-%, particularly preferably 0.2 to 3 wt.-% of PVA, based on the total amount of the solution,
v) adding the mixture obtained in step iii) to the solution obtained in step iv) to produce particles,
vi) optionally: evaporating the solvent, one of or the solvents used in step i) and ii), and
vii) purifying the particles and subsequently dispersing the particles in a dispersant, preferably in a pharmaceutically acceptable solvent,
viii) providing one, two, three or more surface coating material(s) and coating the particles therewith,
ix) optionally: providing and adding one or more further pharmaceutically acceptable components to the particles
wherein in step v) the amounts of the mixture and of the solution are selected such that a ratio of a least 1 ml of solution per 5 mg of the summed weight of a.1.i) and the anti-cancer agent(s) is achieved, and
wherein the addition in step v) is performed by injection, preferably constant injection, preferably at a rate in a range of from 0.5 to 5 ml/min, preferably in a range of from 0.5 to 3.0 ml/min, particularly preferably at a rate in a range of from 0.4 to 2.5 ml/min.

12. Method according to claim 11, wherein the solvent used in step i) and/or the solvent used in step ii) comprises or consists of one or more substances selected from the group consisting of acetone ethyl acetate, chlorinated solvents, DMSO, methylated solvents, tetrahydrofuran, halogenated hydrocarbons such as chloroform and dichloromethane, dioxanes, acetonitrile,
and/or
wherein the solvent used in step iv) for providing a PVA solution comprises or consists of one or more substances selected from the group consisting of water, ethanol and saline,
and/or
wherein the dispersant used in step vii) comprises or consists of one or more substances selected from the group consisting of saline, glucose, pH modifiers and water.

13. Method according to one of claims 11 or 12, wherein the one or further pharmaceutically acceptable components is/are selected from the group consisting of carriers, polymers, wetting agents, emulsifiers, antioxidants, pH influencing agents, disintegrants, recrystallization agents, fluxing agents, preservatives, solvents, salts, fillers, binders, foamers, defoamers, lubricants, adsorbents, substances for adjusting the osmotic pressure and buffers.
